Europäisches Patentamt

European Patent Office

Office européen des brevets

(19)

(11) Publication number: **0 311 983 B1**

# EUROPEAN PATENT SPECIFICATION

(12)

(45) Date of publication of patent specification: **29.07.92**   (51) Int. Cl.5: **B01J 29/04**, C01B 33/20

(21) Application number: **88116870.2**

(22) Date of filing: **11.10.88**

---

(54) **Method for the preparation of titanium-silicalites.**

---

(30) Priority: **12.10.87 IT 2222087**

(43) Date of publication of application:
**19.04.89 Bulletin  89/16**

(45) Publication of the grant of the patent:
**29.07.92 Bulletin  92/31**

(84) Designated Contracting States:
**BE DE FR GB IT NL**

(56) References cited:
EP-A- 0 132 550       EP-A- 0 183 391
EP-A- 0 208 311       WO-A-85/04853
US-A- 3 329 481       US-A- 4 246 139
US-A- 4 410 501

(73) Proprietor: **ENICHEM ANIC S.r.l.**
**55, Via Ruggiero Settimo**
**I-90139 Palermo(IT)**

(72) Inventor: **Padovan, Mario**
**1, via Villa Mirabello**
**I-20100 Milan(IT)**
Inventor: **Leofanti, Guiseppe**
**4, p.zza 5 Giornate**
**I-20100 Arese (MI)(IT)**
Inventor: **Roffia, Paolo, Dr.**
**25, via Valletta**
**I-21047 Saronno (VA)(IT)**

(74) Representative: **Barz, Peter, Dr. et al**
**Patentanwälte Dr. V. Schmied-Kowarzik**
**Dipl.-Ing. G. Dannenberg Dr. P. Weinhold Dr.**
**D. Gudel Dipl.-Ing. S. Schubert Dr. P. Barz**
**Siegfriedstrasse 8**
**W-8000 München 40(DE)**

## Description

The invention concerns a method for the preparation of titanium-silicalites, suited for the catalysis of many organic syntheses, in particular the ammoximation in the liquid phase of carbonyl compounds (see EP-A-208 311) and the synthesis of propylene oxide (see EP-A-200 260).

US-A-3 702 886 discloses a new class of alumino-silicates, prepared by hydrothermal (or, by other denomination, "isothermal") synthesis, in the presence of a templating agent, which alumino-silicates show a zeolitic structure, nowadays referred to as PENTASIL structure because of the geometrical shape of the secondary structural units; these alumino-silicates mainly consist of ZSM-5 zeolites. More recent catalysts completely consist of crystalline silicas (substantially aluminum free), having a zeolitic structure of the PENTASIL type, similar to the structure of the ZSM-5 zeolites, as disclosed in US-A-4 073 865 and 4 061 724, these particular silicas being referred to as "silicalites". Still more recently US-A-4 410 501 disclosed a synthetic, crystalline and porous catalytic material, containing titanium, silicon and oxygen in a combined form and having a structure similar to the structure of the silicalite, wherein titanium is present in the structure as a partial substituent for silicon; in said US-A this kind of material is clearly indicated by the expression "titanium-silicalite" and the same term is used in a large number of other patent documents as well (EP-A-132 550; 190 609; 208 311; and 267362).

The preparation of the titanium-silicalites was based, until now, on the initial formation of a gel dispersed in an aqueous solution, followed by a heat treatment in an autoclave under autogenous pressure (hydrothermal synthesis); as the silicon source use was made of a tetra-alkyl-ortho-silicate, of silica in the colloidal form or of an alkali metal silicate and as the titanium source use was made of hydrolyzable compounds, selected preferably from $TiCl_4$, $TiOCl_2$ and $Ti(alkoxy)_4$. Said preparations required, however a high $H_2O:SiO_2$ ratio, as well as a high (templating agent):$SiO_2$ ratio and the reactants had to be heated at a very high temperature for a very long time. There has now been found an alternative, very simple and reliable method, which allows to prepare a titanium-silicalite while lowering in a drastic way the $H_2O:SiO_2$ ratio and therefore the amount of the reactants, as well as the volume of the apparatus.

The invention, in its broadest aspect, resides in a method for the preparation of titanium-silicalites, in the presence of a templating agent, wherein a $TiO_2$-$SiO_2$ coprecipitated material, amorphous or having a crystalline structure and containing titanium, silicon and oxygen in combined form, is impregnated (preferably "dry-impregnated") with an aqueous solution of said templating agent and in that the thus impregnated material which shows certain molar ratios of its components (see below) is made to acquire the zeolitic structure of the titanium-silicalites by means of a hydrothermal synthesis as described below.

The above $TiO_2$-$SiO_2$ coprecipitates, which are also known as "$TiO_2$-$SiO_2$ binary oxides" or as "$TiO_2$-$SiO_2$ mixed oxides" are solid compounds obtained by bringing into contact, under precipitation conditions, at least two solutions (in water and/or in another solvent) containing soluble compounds of titanium, silicon, and oxygen; the composition and morphologic structure of these compounds may greatly change depending on the reactants concentration, on the solution pH, on the solvent nature and on other coprecipitation conditions. Examples concerning the preparation of these coprecipitates are given in JOURNAL OF CATALYSIS 35, 225-231 (1974) and the literature referred to therein. The possible crystalline structure of said $TiO_2$-$\overline{SiO_2}$ coprecipitates, obviously, must be different from the crystalline structure of the derivative, i.e. of the titanium-silicalites.

By operating in such a way the hydrothermal synthesis is carried out in the presence of lower amounts of templating agent and at a lower temperature, with respect to the usual methods. The present method allows to avoid the formation of a gel dispersed in the liquid phase and therefore the synthesis time is lowered in a drastic way; moreover it allows to use, as the silicon and titanium sources, solid $TiO_2$-$SiO_2$ coprecipitates, which can be easily obtained or are commercially available.

The $TiO_2$-$SiO_2$ coprecipitates generally show a composition range, expressed as $SiO_2:TiO_2$ molar ratio, from 20 to 200. Excellent results were obtained by using $TiO_2$-$SiO_2$ commercial coprecipitates showing a $SiO_2$-$TiO_2$ molar ratio from 30 to 80. The $TiO_2$-$SiO_2$ coprecipitates having a high pore volume and a high surface area, for instance a pore volume higher than 0.5 $cm^3/g$ and a surface area higher than 200 $m^2/g$, proved to be particularly suitable. These coprecipitates are generally available as a powder or in the form of pellets (average size = about 1-3 mm).

When starting from powders, the average size of the obtained titanium-silicalites ranges from 0.2 to 0.5 $\mu$m.

The $TiO_2$-$SiO_2$ coprecipitates, preferably dried at 100-300°C, are impregnated with an aqueous solution of the templating agent; excellent results were obtained by using the dry impregnation technique described, for instance, in IND. ENG. CHEM. PROD. RES. DEV. 20, 441 (1981).

As it is known, there are numerous templating agents which can give rise to zeolites, silicalites and titanium-silicalites showing a PENTASIL structure; see, for instance, la Chimica e l'Industria (Vol. 67 N. 1-2; January-February 1985). Merely exemplary for said templating agents are amines, organic nitro-compounds, alkylene glycols and alkanolamines; use is generally made of tetra-propyl ammonium hydroxide or bromide. The thus impregnated solid is then transferred into an autoclave and it is kept there at 130-170°C for 2 to 20 days, preferably 3 to 11 days. When the synthesis is over, the solid is rinsed with $H_2O$ until a neutral pH is obtained; afterwards it is dried, for instance at 120°C, and calcined, for instance at 430°C. Alternatively, the product may be subjected, after drying and before calcination (or after calcination), to an "activating" washing based on hydrogen peroxide, optionally in the presence of an inorganic acid, as described, for instance, in EP-A-267362. The composition of the material to be subjected to the hydrothermal synthesis, expressed as molar ratio of the reactants, is as follows:

|  |  | preferably |
|---|---|---|
| $SiO_2 : TiO_2$ | 20-200 | 30-80 |
| $OH^- : SiO_2$ | 0.03-0.20 | 0.05-0.10 |
| $H_2O : SiO_2$ | 0.10-10 | 0.50-4 |
| $R_4N^+ : SiO_2$ | 0.03-0.20 | 0.05-0.10 |
| $R_4N^+$ being the organic cation corresponding to the templating agent used. | | |

The X-ray diffractometric analysis shows that the compound obtained according to the present method is crystalline and is characterized by the typical reflexes of the titanium-silicalite, reported in US-A-4,410,501. In figure 1 a typical diffractogram is shown, obtained from the product prepared according to the process described in Example 1, by using the $K\alpha$ radiation of copper. The presence of titanium as a partial substituent for silicon, in the crystalline zeolitic structure, was confirmed by the presence, in the infrared (I.R.) spectrum, of a characteristic absorption band having a maximum substantially at 950 $cm^{-1}$. This band, as shown in US-A-4,410,501, does not appear in the I.R. spectrum of the pure silicalite, free from titanium, or in the mechanical mixtures of metal-silicates and of titanium dioxide described US-A-3,941,871.

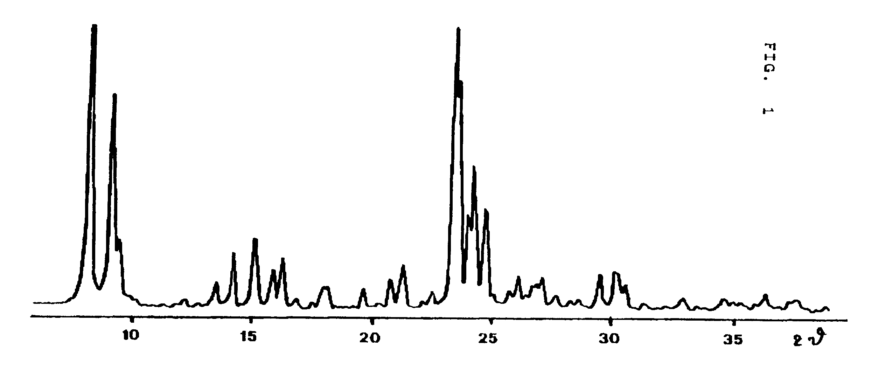

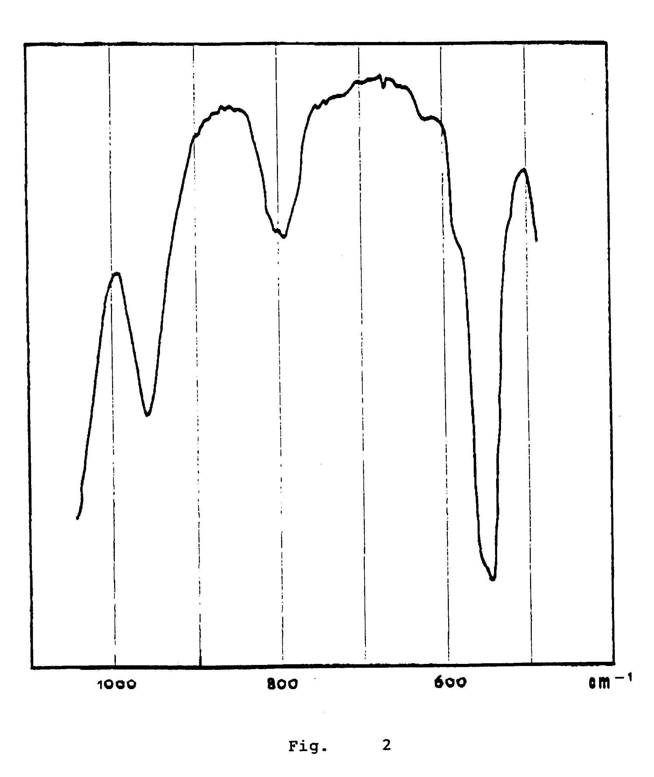

In figure 2 an I.R. spectrum is set forth, which is typical of the compound obtained according to the process described in example 1. The measurement of the band intensity at 950 $cm^{-1}$ on a large number of samples of well crystallized titanium-silicalites having a different titanium content allowed to determine the numerical factor to be used for the calculation of the titanium concentration, just starting from the intensity of the band at 950 $cm^{-1}$. The determination of the absorption isotherm of nitrogen, according to the known BET method, allowed to ascertain that the volume of the intercrystalline cavities was 0.170-0.185 $cm^3/g$, typical of the crystalline solids having a structure of the PENTASIL type, just as the titanium-silicalites. The titanium-silicalites prepared according to the invention and in which the molar ratio $Ti/(Ti+Si)$ ranged from 0.0120 to 0.0190, were successfully used for the ammoximation of carbonyl compounds such as, for instance, acetone, cyclohexanone, methyl-ethyl-ketone (butan-2-one), acetophenone and cyclo-dodecanone, as well as for the oxidation of propylene to propylene oxide.

The ammoximation may be carried out at 25-100 °C (preferably 30-90°C, in particular 60-90°C). The reaction may be carried out at atmospheric pressure or at a pressure slightly higher than atmospheric, in order to maintain the $NH_3$, in amounts at least equal to the synthesis requirement, dissolved in the reaction medium. The molar ratio $H_2O_2$: carbonyl compound may generally range from 0.3 to 2.5 and preferably from 0.5 to 1.3, where $H_2O_2$ denotes pure (100%) hydrogen peroxide (any dilution water excluded). The molar ratios $NH_3$: ketone, carbonyl compound: $NH_3$ and $NH_3$:$H_2O_2$ are preferably equal to or higher than 1 (even better higher than 1.5), otherwise parallel reactions can occur. The reaction medium may consist of water or of an organic solvent; tert-butanol and/or cyclohexanol, optionally in admixture with dioxane or toluene, proved to be particularly suitable solvents. The molar ratio solvent: carbonyl compound may generally range from 0.1 to 100.

The following examples illustrate the invention, without limiting however in any case its scope.

Example 1

20 g of an (amorphous) $TiO_2$-$SiO_2$ coprecipitate, available from GRACE Company as GRACE Silica-Titania No. 1 TYPE I, containing 2.6% by weight of Ti, having a surface are (BET) of 467 $m^2/g$ and a pore volume of 1.07 $cm^3/g$, were dried at 300°C for 4 hours; thereafter said material was dry-impregnated with 20 ml of an aqueous solution of tetrapropyl-ammonium hydroxide at 20% by weight. The thus impregnated solid material was directly transferred into a 50 ml autoclave, where the hydrothermal synthesis was carried out at 150°C for 10 days, without stirring. Then the solid product was rinsed with deionized $H_2O$ until neutral, dried at 120°C for 15 hours and calcined at 430°C for 10 hours. The molar ratio Ti : (Ti + Si), in the end product, was 0.0188, according to the elemental analysis, and 0.0183 according to the analysis by infrared (I.R.) spectrophotometry. The difference between the two values was not higher than the overall experimental error of the two methods. The volume of the intercrystalline cavities, measured by nitrogen absorption, was 0.182 $cm^3$/g, corresponding to a crystallinity degree higher than 95%. The average size of the obtained particles was 0.5 $\mu$m.

Example 2 (application example)

A glass reactor, equipped with a stirrer and heating jacket, was pressurized with an inert gas (nitrogen). Then 1.5 g of a fine powder of titanium-silicalite, obtained according to example 1 were loaded into the reactor. One added then 21 g of water (1.17 moles), 25 g of t-butyl alcohol (0.34 moles) and 4 g of ammonia (0.24 moles); after having put the whole under stirring, 10.34 g of cyclohexanone (0.105 moles) were loaded, thereby forming a biphasic (solid-liquid) system, that was kept homogeneous by powerful stirring. The temperature was gradually increased up to 80°C, by conveying a thermostatic liquid into the reactor jacket. One started then to feed into the reactor, by means of a metering pump, an aqueous solution of hydrogen peroxide at 27.4% by weight. During the heating, the pressure rose slightly above the atmospheric level.

The addition of hydrogen peroxide was continued over 5 hours and 11.33 of $H_2O_2$ (0.096 moles) were fed (on the whole); during the addition a pressure drop was noted.

After cooling, one added to the thus obtained suspension ethyl ether and one stirred for a few minutes; then the aqueous and ether phases were separated from the catalyst by filtration. The liquid phases were separated (in a separatory funnel) and the gaschromatographic analysis showed an 85.4% cyclohexanone conversion and a 95.9% selectivity to oxime; 6.4% of $H_2O_2$ was lost because of decomposition and the oxime yield (with respect to $H_2O_2$) was 90.0%.

Example 3 (Activating washing of the catalyst with $H_2O_2$).

10 g of the titanium-silicalite prepared according to example 1 were soaked (at 70°C) in an aqueous solution, prepared by mixing, under stirring, 20 ml of aqueous $H_2O_2$ (at 30% by weight) with 177 ml of aqueous $H_2SO_4$ at 10% by weight; after 2 hours the liquid was separated by decantation. This activating washing (with $H_2O_2$ and $H_2SO_4$) was repeated twice with fresh solutions, whereafter a filtration was carried out, followed by a long rinsing with deionized $H_2O$ (until neutral). The rinsed solid was dried at 120°C for 15 hours and finally calcined in air at 550°C for 2 hours. The molar ratio Ti:(Ti + Si), in the end product was 0.0167 according to the elemental analysis. The volume of the intercrystalline cavities, measured by nitrogen absorption, was 0.183 $cm^3$/g.

Example 4 (application example)

Example 2 was repeated,replacing the former catalyst by a catalyst activated by a treatment with $H_2SO_4$ and $H_2O_2$ as described in example 3; the experimental procedure was the same, whereas the amounts of the reactants were: 10.42 g of cyclohexanone (0.106 moles) and 11.67 g of $H_2O_2$ at 2.4% (0.094 moles). One obtained the following results:

| | |
|---|---|
| Cyclohexanone conversion | 83.8% |
| Selectivity of cyclohexanone to oxime | 99.7% |
| Oxime yield (with respect to $H_2O_2$) | 94.4% |
| Hydrogen peroxide loss | 2.5% |

Example 5

Example 3 was repeated, replacing the basic coprecipitate by an amorphous coprecipitate available from GRACE Company as Silica-Titania No. 2 Type III, containing 1.38 by weight of titanium, having a surface area (BET) of 342 $m^2$/g and a pore volume of 1.21 $cm^3$/g. The product obtained from the hydrothermal synthesis, activated by a treatment with $H_2SO_4$ and hydrogen peroxide, showed a molar ratio Ti:(Ti + Si) of 0.0122 according to the elemental analysis. The volume of the intercrystalline cavities, measured by nitrogen absorption, was 0.184 $cm^3/g$, corresponding to a crystallinity degree of 98%; the average size of the obtained particles was about 0.5 $\mu$m.

Example 6 (application example)

Example 2 was repeated, replacing the former catalyst by a catalyst prepared according to the procedure described in example 5 (starting from a different $TiO_2$-$SiO_2$ matrix), activated, after the hydrothermal synthesis, by a treatment with $H_2SO_4$ and hydrogen peroxide; the amounts of the reactants were 9.51 g of cyclohexanone (0.097 moles) and 10.25 g of $H_2O_2$ at 31.5% (0.095 moles). The obtained results were:

| | |
|---|---|
| Cyclohexanone conversion | 88.8% |
| Selectivity of cyclohexanone to oxime | 100% |
| Oxime yield (with respect to $H_2O_2$) | 91.7% |
| Hydrogen peroxide loss | 7.5% |

The results of the examples 2,4 and 6 are summarized in Table 1.

TABLE 1

| EXAMPLE | 2 | 4 | 6 |
|---|---|---|---|
| Catalyst | From ex.1 | From ex.3 | From ex.5 |
| Ketone conversion (%) | 85.4 | 83.8 | 88.8 |
| Selectivity of ketone to oxime (%) | 95.9 | 99.7 | 100 |
| Oxime yield, with respect to $H_2O_2$ (%) | 90.0 | 94.4 | 91.7 |
| $H_2O_2$ loss (%) | 6.4 | 2.5 | 7.5 |

## Claims

1. A method for the preparation of titanium-silicalites in the presence of a templating agent, wherein a coprecipitated $TiO_2$-$SiO_2$ material, amorphous or having a crystalline structure and containing titanium, silicon and oxygen in combined form, is impregnated with an aqueous solution of said templating agent and the thus impregnated material which shows the following molar ratios:
   $SiO_2/TiO_2$ = from 20 to 200
   $OH^-/SiO_2$ = from 0.03 to 0.20
   $H_2O/SiO_2$ = from 0.10 to 10
   $R_4N^+/SiO_2$ = from 0.03 to 0.20
   $R_4N^+$ being the nitrogen containing organic cation corresponding to the templating agent used;
   is subjected to a hydrothermal synthesis comprising treatment at 130 to 170°C in an autoclave for 2 to 20 days, subsequent washing with water until neutral, drying and calcination.

2. A method according to claim 1, wherein said coprecipitated material is amorphous.

3. A method according to claim 1 or 2, wherein the pore volume of the coprecipitate is higher than 0.5 $cm^3/g$, the surface area being higher than 200 $m^2/g$.

4. A method according to anyone of the preceding claims, wherein the $SiO_2$:$TiO_2$ molar ratio in said coprecipitate ranges from 20 to 200 and preferably from 30 to 80.

5. A method according to anyone of the preceding claims, wherein said coprecipitate is impregnated according to the dry-impregnation technology.

6. A method according to anyone of the preceding claims, wherein the titanium-silicalite is subjected, before being used, to an activating washing based on hydrogen peroxide, optionally in the presence of an inorganic acid.

5

**7.** A method according to anyone of the preceding claims. wherein said templating agent is selected from tetrapropylammonium hydroxide and tetrapropylammonium bromide.

**8.** A method according to claim 1, wherein the material to be subjected to the hydrothermal synthesis shows the following molar ratios:

$SiO_2/TiO_2$ = from 30 to 80
$OH^-/SiO_2$ = from 0.05 to 0.10
$H_2O/SiO_2$ = from 0.50 to 4
$R_4N^+/SiO_2$ = from 0.05 to 0.10.

## Revendications

**1.** Un procédé pour la préparation de silicalites de titane en présence d'un agent formant un gabarit, dans lequel un matériau coprécipité de type $TiO_2$-$SiO_2$ amorphe ou ayant une structure cristalline et contenant du titane, du silicium et de l'oxygène sous une forme combinée, est imprégné avec une solution aqueuse de cet agent formant un gabarit et le matériau ainsi imprégné, qui présente les rapports molaires suivants :

$SiO_2/TiO_2$    de 20 à 200
$OH^-/SiO_2$    de 0,03 à 0,20
$H_2O/SiO_2$    de 0,10 à 10
$R_4N^+/SiO_2$    de 0,03 à 0,20

$R_4N^+$ étant le cation organique correspondant à l'agent formant un gabarit utilisé;
est soumis à une synthèse hydrothermique comprenant un traitement entre 130 170°C dans un autoclave pendant 2 à 20 jours, un lavage ultérieur à l'eau jusqu'à neutralité, un séchage et une calcination.

**2.** Un procédé suivant la revendication 1, dans lequel ce matériau coprécipité est amorphe.

**3.** Un procédé suivant la revendication 1 ou 2, dans lequel le volume de pore du coprécipité est supérieur à 0,5 cm$^3$/g et l'aire superficielle est supérieure à 200 m$^2$/g.

**4.** Un procédé suivant l'une quelconque des revendications précédentes, dans lequel le rapport molaire $SiO_2/TiO_2$ dans ce coprécipité est de l'ordre de 20 à 200 et de préférence de 30 à 80.

**5.** Un procédé suivant l'une quelconque des revendications précédentes, dans lequel ce coprécipité est imprégné selon la technologie d'imprégnation à sec.

**6.** Un procédé suivant l'une quelconque des revendications précédentes, dans lequel la silicalite de titane est soumise, avant utilisation, à un lavage activant à base de peroxyde d'hydrogène, éventuellement en présence d'un acide inorganique.

**7.** Un procédé suivant l'une quelconque des revendications précédentes, dans lequel cet agent formant un gabarit est choisi parmi l'hydroxyde de tétrapropylammonium et le bromure de tétrapropylammonium.

**8.** Un procédé suivant la revendication dans lequel le matériau devant être soumis à la synthèse hydrothermique présente les rapports molaires suivants :

$SiO_2/TiO_2$    de 30 à 80
$OH^-/SiO_2$    de 0,05 à 0,10
$H_2O/SiO_2$    de 0,50 à 4
$R_4N^+/SiO_2$    de 0,05 à 0,10.

## Patentansprüche

**1.** Verfahren zur Herstellung von Titansilikaliten in Gegenwart einer Templatverbindung, worin ein copräzipitiertes $TiO_2$-$SiO_2$-Material, das amorph oder von kristalliner Struktur ist und Titan, Silicium und Sauerstoff in kombinierter Form enthält, mit einer wäßrigen Losung der Templatverbindung imprägniert wird und das imprägnierte Material, welches die folgenden Molverhältnisse aufweist:

$SiO_2/TiO_2$ = 20 bis 200
$OH^-/SiO_2$ = 0,03 bis 0,20
$H_2O/SiO_2$ = 0,10 bis 10
$R_4N^+/SiO_2$ = 0,03 bis 0,20,

wobei $R_4N^+$ das stickstoffhaltige organische Kation ist, welches der verwendeten Templatverbindung entspricht;

einer hydrothermalen Synthese unterworfen wird, welche eine 2- bis 20tägige Behandlung in einem Autoklaven bei 130 bis 170°C, anschließendes Waschen mit Wasser bis zur Neutralreaktion, Trocknen und Calcinieren umfaßt.

2. Verfahren nach Anspruch 1, worin das copräzipitierte Material amorph ist.

3. Verfahren nach Anspruch 1 oder 2, worin das Porenvolumen des Copräzipitats größer als 0,5 cm$^3$/g ist, wobei die Oberfläche größer als 200 m$^2$/g ist.

4. Verfahren nach irgendeinem der vorangehenden Ansprüche, worin das Molverhältnis $SiO_2/TiO_2$ in dem Copräzipitat 20 bis 200, vorzugsweise 30 bis 80, beträgt.

5. Verfahren nach irgendeinem der vorangehenden Ansprüche, worin das Copräzipitat mittels einer Trockenimprägniertechnik imprägniert wird.

6. Verfahren nach irgendeinem der vorangehenden Ansprüche, worin das Titansilikalit vor der Verwendung einer Aktivierungswaschung auf Basis von Wasserstoffperoxid, gegebenenfalls in Gegenwart einer anorganischen Säure, unterzogen wird.

7. Verfahren nach irgendeinem der vorangehenden Ansprüche, worin die Templatverbindung ausgewählt ist unter Tetrapropylammoniumhydroxid und Tetrapropylammoniumbromid.

8. Verfahren nach Anspruch 1, worin das der hydrothermalen Synthese unterzogene Material die folgenden Molverhältnisse aufweist:
$SiO_2/TiO_2$ = 30 bis 80
$OH^-/SiO_2$ = 0,05 bis 0,10
$H_2O/SiO_2$ = 0,50 bis 4
$R_4N^+/SiO_2$ = 0,05 bis 0,10.

7

FIG. 1

Fig.    2